# EUROPEAN PATENT SPECIFICATION

(11) **EP 2 804 644 B1**
(45) Date of publication and mention of the grant of the patent: **01.05.2019**
(21) Application number: 13736304.0
(22) Date of filing: 09.01.2013
(51) Int. Cl.: A61M 5/14, F16M 11/20

(54) **A STORAGE SYSTEM FOR A PORTABLE IV-STAND**
LAGERUNGSSYSTEM FÜR EINEN TRAGBAREN IV-STÄNDER
SYSTÈME DE RANGEMENT POUR PIED À PERFUSIONS PORTABLE

(30) Priority: 10.01.2012 SE 1200026
(43) Date of publication of application: 26.11.2014
(73) Proprietor: Tarsus Products AB, 891 50 Örnsköldsvik (SE)
(72) Inventor: SJÖBLOM, Olow, S-891 50 Örnsköldsvik (SE)
(86) International application number: PCT/SE2013/000003
(87) International publication number: WO 2013/105889

(56) References cited:
- DE-U1- 8 810 990
- DE-U1- 8 810 990
- DE-U1- 9 213 242
- DE-U1-202009 015 855
- SE-A1- 1 000 548
- SE-A1- 1 000 548
- US-A- 4 744 536
- US-A- 4 892 279
- US-A- 5 839 586
- US-A1- 2005 247 836
- US-A1- 2005 269 464
- US-A1- 2005 269 464
- US-A1- 2006 065 613

## Description

### Field of the Invention

The present invention concerns an Iv stand (stand for intravenous bags) and a system for storing Iv stands in accordance with the claims.

### Background of the Invention

In medical care a large number of intravenous infusions containing for example drip, blood and the like are administered on a daily basis. Intravenous infusions are usually carried out by transferring fluids from so-called Iv bags (infusion bags) to a patient's bloodstream via an intravenous line (Iv line) or similar. The transference of fluids from Iv bags to the patient occurs normally when the Iv bag is hung on a so called Iv stand at a height above the patient, allowing for the force of gravity to transfer the fluid to the patient. Alternatively, the fluid in the Iv bag may be transferred to the patient by pumping the fluid with an infusion pump or similar.

Iv stands usually include a vertical pole, a suspension device for bags and a base, foot or similar. The vertical pole's length is telescopically adjustable in order to satisfy the need of adjusting the height of the bag relative the patient. Existing designs therefore usually include a first pole section and a second pole section which are telescopically adjustable relative each other. The fact that the pole is usually in two sections allows the stand, only to a limited extent, to be shortened in the stand's longitudinal direction.

One problem with existing types of Iv stands is that they usually require considerable space both in usage and storage when not in use. This problem is especially problematic in conjunction with storage of Iv stands when not in use. A problem with existing Iv stands is that they usually arc designed to stand on the floor or the like. This means that the storage of multiple Iv stands is usually disordered in nature, with stands easily hooking into each other, and that the storage of Iv stands requires considerable space.

In order to reduce the need for space required to store Iv stands, the base feet of Iv stands have been designed to be collapsible (fold together). This has allowed stands to take up less space but the storage of stands is still disorderly in nature. Therefore there is a need for a substantially improved Iv stand which takes up minimal space and with simplicity may be stored together in large numbers simultaneously. According to the present invention, this problem may be solved by hanging up the Iv stands when not in use. The problem is that existing types of Iv stands are not designed to be hung up and therefore lack a suspension device with which the stand may be hung up when not in use.

Another problem with existing types of Iv stands is that the locking devices between the stands' telescopic sections may be difficult to adjust quickly. The problem is brought about by the fact that there may exist inertia between the included parts of the Iv stand.

A still further problem with existing types of Iv stands is that they are strenuous to fold together. It may be difficult for a user, especially users with week muscles to, for example fold out and fold together the base of the Iv stand. A yet further problem with existing designs of Iv stands is that they include a large number of details and therefore are costly to manufacture.

### Prior Art

A large number of variants of stands for Iv bags are already known. For example, US4892279 describes a variant of a stand for Iv bags. The design includes a base whose legs may be folded in (retracted). The design also includes a three-piece telescoping pole. The design differs substantially from the present invention's design. For example, the design according to its description lacks a suspension device in accordance with the present invention's design.

In US5458305 is described a further variant of a stand for Iv bags with a folding (collapsible) base. The design further includes a telescopically arranged three-piece pole. The stand according to its description lacks a suspension device, with which the stand may be hung up, in a similar manner as with the present invention's design. Therefore, this design differs substantially from the design according to the present invention.

In US4744536 is described a variant of an Iv stand including a collapsible base, a telescopically adjustable pole and two pivotally attached hooks The design of US4744536, according to its description, may only however be hung upon an object with an open (freed) end which limits the possibilities of hanging up the Iv stand. The suspension device in accordance -with the present patent application may be hung upon a rod (bar) or similar without any of the rod's ends or similar being open (free) at one of its ends. This is achieved by the suspension device being made of hooks that are pivotal around a common center of rotation and that they may be rotated around (and away from) a rod or similar in conjunction with the Iv stand being hung up.

In DE8810990U is described a variant of an Iv stand including a base, a pole and two attached suspension devices for suspending two Iv stands. The design oF DESS 10990U, according to its description, may only however have one stand hung on one level which limits the possibilities of hanging up multiple Iv stands on the same level. Therefore, this design differs substantially from the design according to the present invention. For example, the suspension device in accordance with the present patent application allows for several stands to be suspended on the same level. Further, the design of DE8810990U, according to its description, does not allow for the storage of collapsible Iv stands.

Patent application SE1000548 describes a variant of a stand for infusion bags which is comprised of two suspension members that at a first glance are similar to the suspension members in accordance with the present application. The suspension member in accordance with SE536440 however, differs significantly from the suspension member in accordance with the present patent application.

The patent application US2006/065613 describes an article organizer for storing, organizing, and displaying jewelry and similar articles, comprising a shaft and a plurality of storage units mounted around the shaft, which storage units may be individually rotatable. The construction according to the application differs significantly from the construction in accordance with the present patent application.

The patent application US2005/247836 describes a display stand having a shaft extending upwards from a bottom plate. Multiple support bars extending horizontally outward from the top section of the shaft in one or more levels. Each level having a concentric outer support ring and inner support ring connected to each of the multiple support bars. Each level having a plurality of display rods detachably connected to the inner support ring and outer support ring. The design in accordance with US2005247836 however, differs significantly from the design in accordance with the present patent application. For example, the design does not include a system for storage of IV stands.

The patent US5839586 describes an apparatus for organizing and hanging a plurality of IV bags on a carousel for use primarily in a refrigerator which includes a central column having disk spacable there along which are rotatable relative to the central column, the disk further being equipped with a plurality of hangers for organizing IV bags held in refrigerated suspension prior to dispersion to patients. The design in accordance with US5839586 however, differs significantly from the design in accordance with the present patent application. For example, the design does not include a system for storage of IV stands.

In DE9213242U1 an IV stand is described which includes suspension members that pivot, rotate or turn. The design in accordance with DE9213242U1 however, differs significantly from the design in accordance with the present patent application. For example, the design does not include a system for storage of IV stands.

### Brief Description of the Invention Concept

The main purpose of the present invention is to create a substantially improved Iv stand and a system for storing several Iv stands. Another purpose is to create a system that includes a suspension stand in which the Iv stand may be hung upon in at least two vertical levels. A further purpose of the present invention is to create an Iv stand that can be folded together and stored in a more space efficient manner than previously known designs. A yet further purpose of the present invention is to create an Iv stand that may be hung up individually or together with several other stands.

### Detailed Description of the Invention

The present invention will be described in greater detail below with reference to the accompanying schematic drawings that in an exemplifying purpose show the current preferred embodiments of the invention.
Fig. 1A shows a system for storage of Iv stands seen in perspective from the side.
Fig. 1B shows an exemplifying embodiment of a suspension stand for an Iv stand.
Fig. 2 shows an extended (folded out) Iv stand in accordance with the present invention,
Fig. 3 shows a collapsed (folded together) Iv stand in accordance with a first embodiment.
Fig. 4 shows a design detail of a hub included in the Iv stand's base leg supports.

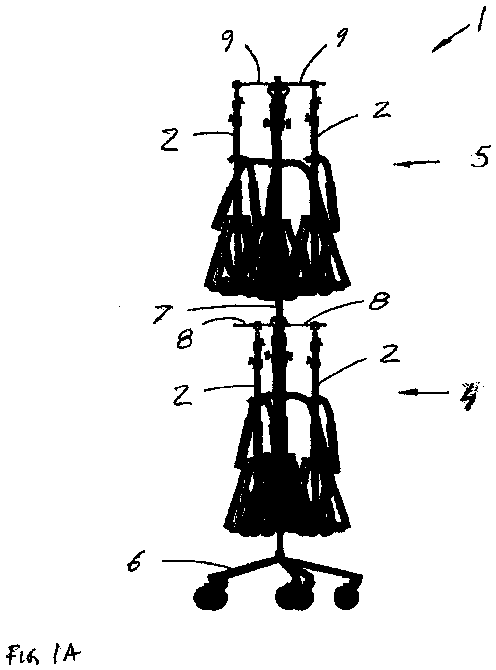

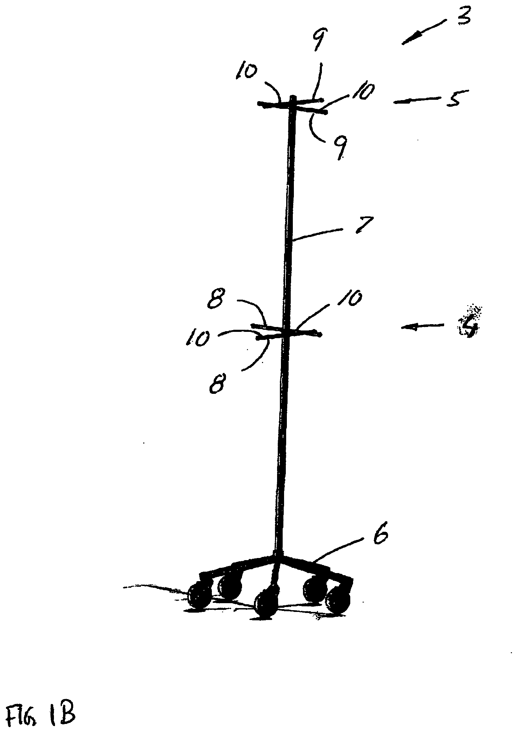

With reference to the figures and more specifically to Fig. 1A is shown a system **1** for storage of Iv stands **2** in accordance with a first embodiment, The meaning of term system 1 is a system for storing at least one first Iv stand 2 and at least one second Iv stand 2 and the Iv stand's design that allows for storage of the Iv stand. In the exemplifying embodiment of the system for storage of Iv stands, the Iv stands 2 are intended, when not used, to be stored hanging in a storage unit or the like. In the exemplifying embodiment, the Iv stands are stored hanging on a suspension stand **3.** Upon the suspension stand 3, the Iv stands are hung up on one first vertical level **4** and one second vertical level **5,** wherein at least one first Iv stand 2 is suspended on at least one first vertical level **4** and at least one second Iv stand 2 is suspended on one second vertical level 5.

In the exemplifying embodiment of the suspension stand 3, it includes a base supporting device **6** to which at least one vertical pole 7 is connected. The base supporting device 6 may be comprised of a foot which is comprised of three or more legs. Each leg may be provided with at least one wheel.

The pole 7 includes at least one suspension member **8** on one first vertical level 4 and at least one suspension member **9** on one second vertical level 5. On each respective first level 4 and second level 5, multiple suspension members 8 and 9 may be connected to the pole 7. Each suspension member 8 and 9 may each be adapted to support one or more Iv stands. For example, each suspension member 8 and 9 may be adapted to suspend one Iv stand, two Iv stands, three Iv stands, four Iv stands and so on. The invention lies in that this is accomplished by the suspension members projecting length from the pole being varied according to the number of Iv stands to be suspended on the suspension member. In the exemplifying embodiment, the suspension member consists of a rod (bar) **10**, pin or other projecting member extending out a ways from the pole. Each respective suspension member 8 and 9, in an alternative embodiment, may include one first part and one second part which are telescopically arranged.

The term Iv stands 2 also concerns designs which are called infusion stands (racks), drip stands and the like. The Iv stand 2 is intended to be used to suspend at least one infusion bag. The Iv stand 2 includes at least one pole **11**, at least one first suspension device **12** for at least one infusion bag and at least one base supporting device **13.**

A unique feature of the Iv stand 2 is that the suspension device 12 for suspending Iv bags may also be used as a suspension device **14** for the Iv stand 2 in for example the suspension stand 3 when the Iv stand 2 is not used. Unique to the preferred embodiment of the Iv stand shown in the figures is that the first suspension device 12 and the second suspension device 14 are integrated in one and the same suspension device.

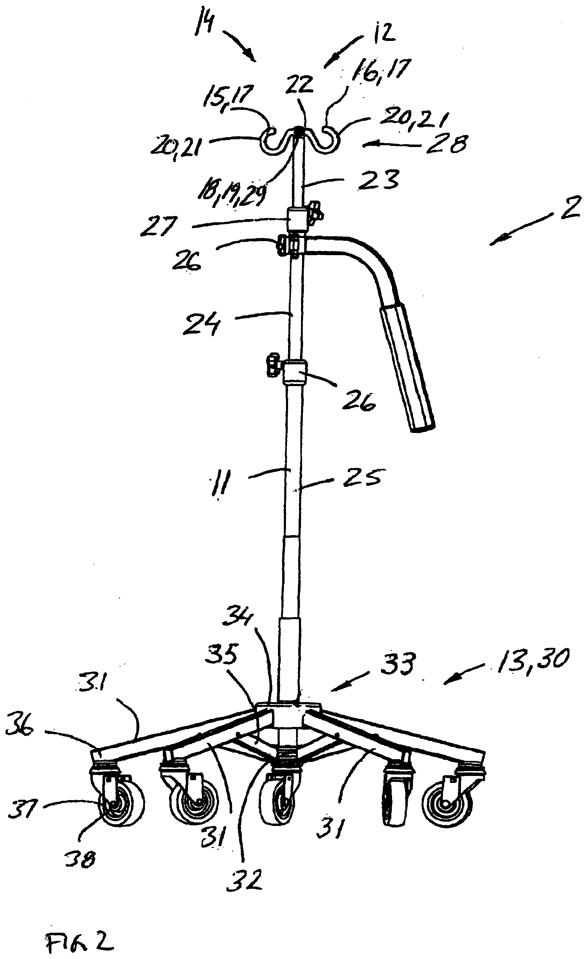

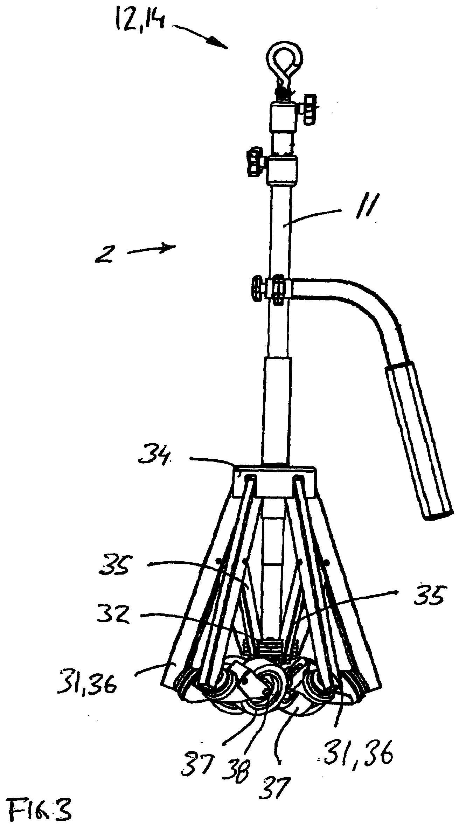

Referring to Fig. 2 and 3, the combined suspension device 12 for the Iv bags and the suspension device 14 for the stand in its entirety is shown in more detail. The suspension device 12 includes at least one first suspension member **15** and preferably at least one second suspension member **16.** The first suspension member 15 and the second suspension member 16 in the exemplifying embodiment consist of a hook-like arrangement (claw-like arrangement) **17.** The hook-like arrangement 17 includes at its one end **18** a fastening eyelet **19** or the like. The second end **20** of the hook-like arrangement 17 is comprised of a curved part **21.** The curved part 21 is preferably radius shaped. In alternative embodiments, the curved part 21 may be of another shape suitable for the purpose. Suspension member 15 and suspension member 16 are pivotally connected to a bracket **22** on the pole 11 via a shaft in the pole's horizontal direction. The bracket 22 includes at least one through hole. The first suspension member 15 and the second suspension member 16 are connected to the bracket 22 by a screw joint, shaft or similar.

A unique feature of suspension members 15 and 16, is that they are positioned on a common axis and that the suspension members are operable between at least one first position where the suspension members are intended to support at least one Iv bag and one second position where the suspension members are rotated in the vertical upward direction towards one another, to one in the vertical direction top position which allows the suspension of the stand 2 in a vertical position which corresponds, or substantially corresponds, to the vertical gravitationally influenced direction. The design entails that the suspended Iv stands do not get entangled with each other, and may be suspended together without significantly hooking or catching on other suspended Iv stands.

The pole 11 is preferably made of a pipe, rod, or the like. Preferably, the length of the pole is adjustable. The length of the pole 11 may be adjusted by the pole being telescopically adjustable. The pole 11 includes at least one first section **23** and at least one second section **24** and preferably at least one third section **25** which are telescopically arranged in relation to each other. The first section 23 is insertable into the second section 24. Alternatively, the second section 24 may be insertable into the first section 23. The second section 24 is insertable into the third section 25. Alternatively, the third section 25 may be insertable into the second section 24. The first section 23 may be locked to the second section 24 with at least one first locking device **26** or the like. The second section 24 may be locked relative to the third section 25 with at least one second locking device **27.** At its upper end **28** the telescopic pole 11 is provided with, or connected to, at least one bracket 22 for the first suspension device 12. The bracket's 22 design may vary greatly within the scope of the present invention. For example, it is conceivable that the first suspension device 12 is detachably connected to the pole with a quick coupling. It is further conceivable that bracket 22 is permanently connected to the upper end of the pole.

Suspension member 15 and suspension member 16 are pivotally arranged around an axis of rotation **29** between a first position and a second position. The first position is the position where the Iv bag is intended to be hung. The hook or hooks, are in this position rotated downward with the hook's opening upward in accordance with the position shown in Fig. 2. The second position is the position where the Iv stand is intended to be hung up. The hook, hooks, are in this position rotated up towards each other according to the position shown in Fig. 3.

With reference to Fig. 4, the base supporting device 13 is shown in more detail. The base supporting device 13 in the exemplifying figures consists of a foot **30.** The foot includes at least three legs **31**, and preferably five legs **31**. The foot 30 further includes at least one in relation to the pole fixed part (hub) **32** and in relation to the pole one movable part (hub) **33**. The fixed part 32 is comprised of a bracket for the pole 11 to the foot 30. The attachment between the pole 11 and the fixed part 32 may be done in a manner suitable for the purpose. The vertical pole 11 is intended to connect permanently or arranged to be removable relative the Iv stand 2. For example, the pole and the fixed part 32 may be connected to each other by means of screw joints or other for the purpose suitable fastening elements. Unique to the base supporting device is that it, unlike previous versions of the stand, includes at least one first mounting plate, at least one second mounting plate and at least one spacer plate between the first plate and the second plate.

Each leg 31 in its first end **34** is journalled (bearing) and pivotally mounted to the movable part 33. Each leg further includes at least one strut **35** which in its one end is connected to the leg 31. In its other end the strut 35 is connected to the fixed part 32 relative the pole.

Each leg 31 includes at its other end **36,** or end's proximity, at least one wheel **37.** The wheel's 37 design may vary greatly within the scope of the present invention. The wheel consists preferably of a wheel of a so-called castor wheel type which is pivotal around a vertical axis **38.** The wheels should also be easily rolled. In alternative embodiments, one or more wheels may include a locking function to prevent the wheel (the wheels) from rolling.

The movable part 33 includes a hole in which the pole is placed. The movable part may be moved along the pole's length between one first (lower) position and one second (upper) position. The movable part is preferably journalled in relative to the pole. This journalled mounting may consist of any for the purpose suitable journalled mounting. In its simplest form the journalled mounting consists of a sliding bearing in the form of shrinking tubing attached to the pole.

The Iv stand's 2 base supporting device includes a movable hub 33 and a fixed hub 32 which are connected with each other via the leg 31 and the strut. The strut is via one first shaft arranged to rotate in relation to the fixed hub 32 and via one second shaft arranged to rotate relative to the leg 31. In a preferred embodiment, the fixed hub 32 includes at least one first lower part, at least one upper part and at least one intermediate part which are provided with a corresponding number of recesses as the number of legs 31. In the preferred embodiment of the present invention, the lower part and the upper part and the intermediate part are disc-shaped. Unique to the present invention is that the shaft is connected to a recess in the intermediate part. The recess for the shaft is positioned substantially transversely in relation to the recess for the legs. The lower part and the upper part lack the recess for the shaft, which means that the shaft is held in the recess when the upper part, the intermediate part and the lower part are assembled together.

In an alternative embodiment of the present invention, it may include at least one handle by which the Iv stand may be carried and guided. The handle may be attached permanently or temporarily to the Iv stand. Preferably, the handle is connected temporarily to the Iv stand's pole 11. The handle may include padding or similar. For example, the handle may include foam or similar material that has been covered with at least one hygienic material layer. The handle allows the Iv stand to quickly and easily be carried along, for example in connection with home care and mobile care.

In the detailed description of the present invention, design details may have been omitted which are apparent to persons skilled in the art. Such obvious design details are included to the extent necessary so that the proper and full performance of the present invention is achieved. For example, components such as screw joints, bearings (bushings) and so on are included to the extent necessary so that an adequate function of the design is obtained in accordance with the present patent application.

Even if certain preferred embodiments have been described in detail, variations and modifications within the scope of the invention may become apparent for specialists in the field of the invention and all such are regarded as falling within the scope of the following claims.

### Advantages of the Invention

The present invention has several advantages. The most obvious is that the system's and Iv stand's design allows for more efficient storage of Iv stands when they are not in use than previously known devices for storing Iv stands. Another advantage is that the stand for Iv bags in accordance with the present invention is easy to use. Yet another advantage of the Iv stand is that it can be folded together (collapsed) so that the stand takes very little space to store. A further advantage of the present Iv stand is that it includes a suspension device with which the stand may be effectively hung up (suspended) with along with several other stands when not in use.

## Claims

1. A combination of collapsible IV stands (2) and a system for storage of said IV stands, said system including at least one suspension stand (3) which is comprised of at least one pole (7) which at its bottom end is connected to at least one base supporting device (6), and that said pole (7) on at least one first vertical level (4) includes at least one suspension member (8) and that said pole on at least one second vertical level (5) includes at least one suspension member (9) on which collapsible IV stands (2) during use are hung, whose each respective IV stand (2) of the combination is comprised of at least one pole (11) which at its bottom end is connected to at least one base supporting device (13) and in its upper end includes a suspension device (12), that includes at least one first suspension member and at least one second suspension member which are pivotally arranged around a common axis of rotation in the pole (11), and that the suspension members are suitable for suspending at least one IV bag in a first position and a second position where the suspension members are rotated in the vertical direction upward toward each other to one in the vertical direction upper position that allows for a suspension of the IV stand (2) wherein that the suspension stand's (3) suspension members (8, 9) consist of projecting members such as a rod, pin or similar where the projecting member's length is adjustable allowing for at least two IV stands to be stored at each vertical level and that the IV stand's base supporting device is comprised of at least one movable hub (33) and one fixed hub (32) which are connected to each other via legs (31) and struts whose struts are moveably arranged in relation to the fixed hub (32) via at least one first axle and pivotally arranged in relation to the leg (31) via a second axle and that the fixed hub (32) is comprised of at least one upper disc-shaped part, one intermediate disc-shaped part and one lower disc-shaped part which are provided with recesses for struts and that the first axle is connected at a recess in the intermediate part, where the recess is placed essentially transversely in relation to the recess for the strut, and that the movable hub (33) is journaled in relation to the pole.

2. A combination of collapsible IV stands and a system for storage of said IV stands in accordance with claim 1 **characterized in that** the first vertical level and the second vertical level each include four suspension members which allow for the suspension of at least four suspension members.

3. A combination of collapsible IV stands and a system for storage of said IV stands in accordance with at least one of the previous claims **characterized in that** the IV stand's (2) suspension members (8, 9) are hook-like.

4. A combination of collapsible IV stands and a system for storage of said IV stands in accordance with at least one of the previous claims **characterized in that** the IV stand's vertical pole (11) is comprised of at least one first section (23), at least one second section (24) and at least one third section (25) which are arranged to be mutually telescopic.

## Patentansprüche

1. Kombination aus zusammenklappbaren Infusionsstativen (2) und einem System zur Lagerung der Infusionsstative, wobei das System mindestens einen Aufhängeständer (3) beinhaltet, der aus mindestens einer Stange (7) besteht, die an ihrem unteren Ende mit mindestens einer Basisträgervorrichtung (6) verbunden ist, und die Stange (7) auf mindestens einer ersten vertikalen Ebene (4) mindestens ein Aufhängeelement (8) beinhaltet und die Stange auf mindestens einer zweiten vertikalen Ebene (5) mindestens ein Aufhängeelement (9) beinhaltet, an dem im Gebrauch zusammenklappbare Infusionsstative (2) aufgehängt sind, deren jeweilige Infusionsstative (2) aus der Kombination von mindestens einer Stange (11) besteht, die an ihrem unteren Ende mit mindestens einer Basisträgervotrichtung (13) verbunden ist und in ihrem oberen Ende eine Aufhängevorrichtung (12) beinhaltet, die mindestens ein erstes Aufhängeelement und mindestens ein zweites Aufhängeelement beinhaltet, die um eine gemeinsame Drehachse in der Stange (11) schwenkbar angeordnet sind, und dass die Aufhängeelemente geeignet sind, mindestens einen Infusionsbeutel in einer ersten Position und einer zweiten Position aufzuhängen, in der die Aufhängeelemente in der vertikalen Richtung zueinander nach oben, in eine in der vertikalen Richtung obere Position gedreht werden, die eine Aufhängung des Infusionsstativs (2) ermöglicht, wobei die Aufhängeelemente (8, 9) des Aufhängeständers (3) aus vorstehenden Elementen bestehen, wie beispielsweise einer Stange, einem Stift oder dergleichen, wobei die Länge der vorstehenden Elemente verstellbar ist, so dass mindestens zwei Infusionsstative in jeder vertikalen Ebene gelagert werden können und dass die Basisträgervorrichtung der Infusionsstative aus mindestens einer beweglichen Nabe (33) und einer festen Nabe (32) besteht, die über Schenkel (31) und Streben, deren Streben über mindestens eine erste Achse beweglich in Bezug auf die feste Nabe (32) und über eine zweite Achse schwenkbar in Bezug auf den Schenkel (31) angeordnet sind, miteinander verbunden sind, und dass die feste Nabe (32) aus mindestens einem oberen scheibenförmigen Teil, einem scheibenförmigen Zwischenteil und einem unteren scheibenförmigen Teil besteht, die mit Aussparungen für Streben versehen sind, und dass die erste Achse an einer Aussparung im Zwischenteil verbunden ist, wobei die Aussparung im Wesentlichen quer zur Aussparung für die Strebe angeordnet ist, und dass die bewegliche Nabe (33) in Bezug zur Stange gelagert ist.

2. Kombination aus zusammenklappbaren Infusionsstativen und einem System zur Lagerung der Infusionsstative nach Anspruch 1, **dadurch gekennzeichnet, dass** die erste vertikale Ebene und die zweite vertikale Ebene jeweils vier Aufhängeelemente beinhalten, die das Aufhängen von mindestens vier Aufhängeelementen ermöglichen.

3. Kombination aus zusammenklappbaren Infusionsstativen und einem System zur Lagerung der Infusionsstative gemäß mindestens einem der vorhergehenden Ansprüche, **dadurch gekennzeichnet, dass** die Aufhängeelemente (8, 9) der Infusionsstative (2) hakenartig sind.

4. Kombination aus zusammenklappbaren Infusionsstativen und einem System zur Lagerung der Infusionsstative gemäß mindestens einem der vorhergehenden Ansprüche, **dadurch gekennzeichnet, dass** die vertikale Stange (11) des Infusionsstativs aus mindestens einem ersten Abschnitt (23), mindestens einem zweiten Abschnitt (24) und mindestens einem dritten Abschnitt (25) besteht, die so angeordnet sind, dass sie gegenseitig ausziehbar sind.

## Revendications

1. Une combinaison de supports pliables pour perfusion (2) et un système de stockage des dits supports pour perfusion, ce système comprenant au moins un support de suspension (3) qui est constitué d'au moins un pied (7) qui est connecté à son extrémité inférieure à au moins un dispositif de support de base (6), et ce pied (7) sur au moins un premier niveau vertical (4) comprend au moins un élément de suspension (8) et ledit pied sur au moins un deuxième niveau vertical (5) comprend au moins un élément de suspension (9) sur lequel les supports pliables pour perfusion (2) sont suspendus durant l'utilisation, dont chaque support pour perfusion (2) respectif de la combinaison est constitué d'au moins un pied (11) qui est connecté à son extrémité inférieure à au moins un dispositif de support de base (13) et à son extrémité supérieure comprend un élément de suspension (12), qui inclut au moins un premier élément de suspension et au moins un deuxième élément de suspension qui sont organisés de manière pivotante autour d'un axe commun de rotation sur le pied (11). Les éléments de suspension sont appropriés pour suspendre au moins un sac de perfusion dans une première position et une seconde position où les éléments de suspension sont tournés dans le sens vertical l'un au-dessus de l'autre, un dans la position verticale supérieure qui permet une suspension du support de perfusion (2) où les éléments de suspension (8, 9) du support de suspension (3) consistent en des éléments de projection tels qu'une tige, broche ou similaire, où la longueur de l'élément de projection est réglable permettant qu'au moins deux supports de perfusion soient stockés à chaque niveau vertical et que le dispositif de support de base du support de perfusion soit constitué d'au moins un moyeu mobile (33) et d'un moyeu fixe (32) qui sont connectés l'un à l'autre par des pieds (31) et d'entretoises dont les entretoises sont disposées de manière mobile par rapport au moyeu fixe (32) par au moins un premier axe et disposé de manière pivotante par rapport au pied (31) via un deuxième axe et que le moyeu fixe (32) est constitué d'au moins une partie supérieure en forme de disque, une partie intermédiaire en forme de disque et d'une partie inférieure en forme de disque qui sont fournies avec des cavités pour entretoises et que le premier axe est connecté à une cavité dans la partie intermédiaire, où la cavité est essentiellement placée de manière transversale par rapport à la cavité pour l'entretoise, et le moyeu amovible (33) est tourillonné par rapport au pied.

2. Une combinaison de supports pliables pour perfusions et un système pour stocker lesdits supports pour perfusion conformément à la revendication 1 **caractérisée par le fait que** le premier niveau vertical et le second comprennent chacun quatre éléments de suspension qui permettent de suspendre au moins quatre éléments de suspension.

3. Une combinaison de supports pliables pour perfusions et un système pour stocker lesdits supports pour perfusion conformément à au moins une des précédentes revendications **caractérisée par le fait que** les éléments de suspension (8, 9) du support pour perfusion (2) sont en forme de crochets.

4. Une combinaison de supports pliables pour perfusion et un système pour stocker lesdits supports pour perfusions conformément à au moins une des précédentes revendications **caractérisée par le fait que** le pied vertical (11) du support pour perfusions est constitué d'au moins une première section (23), au moins une deuxième section (24) et une troisième section (25) qui sont organisées pour être mutuellement télescopiques.
